**Europäisches Patentamt**

**European Patent Office**

**Office européen des brevets**

(19)

(11) Numéro de publication : **0 014 490**

**B1**

(12)

# FASCICULE DE BREVET EUROPÉEN

(45) Date de publication du fascicule du brevet :
13.10.82

(51) Int. Cl.³ : **C 12 P 7/62**

(21) Numéro de dépôt : **80200032.3**

(22) Date de dépôt : **14.01.80**

(54) **Procédé de séparation de poly-bêta-hydroxybutyrates d'une biomasse.**

(30) Priorité : 22.01.79 FR 7901862

(43) Date de publication de la demande :
20.08.80 (Bulletin 80/17)

(45) Mention de la délivrance du brevet :
13.10.82 Bulletin 82/41

(84) Etats contractants désignés :
AT BE CH DE FR GB IT LU NL SE

(56) Documents cités :
FR A 2 338 291
FR A 2 360 667
FR A 2 369 343
US A 3 044 942
US A 3 275 610

(73) Titulaire : SOLVAY & Cie (Société Anonyme)
Rue du Prince Albert, 33
B-1050 Bruxelles (BE)

(72) Inventeur : Vanlautem, Noel
Rue des Drapiers, 17
B-1300 Wavre (BE)
Inventeur : Gilain, Jacques
Avenue de la Ramée, 10
B-1180 Bruxelles (BE)

(74) Mandataire : Elschen, Roland
Solvay & Cie Département de la Propriété Industrielle
Rue de Ransbeek 310
B-1120 Bruxelles (BE)

Imprimerie Jouve, 18, rue St-Denis, 75001 Paris, France

## Procédé de séparation de poly-β-hydroxybutyrates d'une biomasse

La présente invention concerne un procédé de séparation de poly-β-hydroxybutyrates d'une biomasse par extraction au moyen de solvants halogénés liquides.

De nombreux microorganismes sont capables de synthétiser des poly-β-hydroxybutyrates dont la fonction principale dans ces microorganismes semble être le stockage de l'énergie sous forme de matière carbonée. Ces poly-β-hydroxybutyrates constituent une matière première intéressante sur le plan industriel. Diverses techniques ont déjà été envisagées pour les séparer des biomasses. Ainsi, on a déjà proposé de les extraire de la biomasse au moyen de solvants halogénés liquides à bas point d'ébullition tels que le chloroforme et le chlorure de méthylène. Dans ces procédés connus, l'extraction est généralement réalisée à la température normale d'ébullition du solvant ou à une température inférieure. Ainsi réalisée, l'extraction nécessite des durées de traitement très longues et est accompagnée d'une dépolymérisation des poly-β-hydroxybutyrates bien que l'on opère à basse température.

Dans le but de réduire les durées d'extraction et d'augmenter les rendements, on a proposé de mettre en œuvre des solvants tels que le carbonate cyclique de 1,2-éthanediol (carbonate d'éthylène) ou de 1,2-propanediol (carbonate de propylène) à des températures plus élevées. Ces procédés permettent effectivement d'atteindre les buts visés mais ils engendrent une dépolymérisation importante des poly-β-hydroxybutyrates.

La présente invention vise à fournir un procédé qui ne présente plus aucun des inconvénients des procédés connus.

La présente invention concerne à cet effet un procédé de séparation de poly-β-hydroxybutyrates d'une biomasse par extraction au moyen de solvants halogénés liquides, selon lequel on utilise des solvants halogénés liquides choisis parmi les chloroéthanes et les chloropropanes.

Par chloroéthanes et chloropropanes, on entend désigner tous les composés dérivés de l'éthane ou du propane dans lesquels un ou plusieurs atomes d'hydrogène a ou ont été substitué(s) par l'atome de chlore ainsi que les chloroéthanes et les chloropropanes dont certains atomes d'hydrogène ont en outre été substitués par des atomes d'halogène tels que l'iode et le brome et, de préférence, le fluor. On préfère toutefois utiliser des chloroéthanes et des chloropropanes qui ne sont pas substitués et qui comportent dans la molécule uniquement des atomes de chlore, de carbone et d'hydrogène.

Le milieu liquide utilisé pour l'extraction selon l'invention, bien que constitué essentiellement de chloroéthanes et de chloropropanes, peut contenir de faibles quantités d'autres liquides. On préfère cependant qu'il soit constitué de chloroéthanes et de chloropropanes à raison de 90 % en poids, et plus particulièrement 99 % en poids, au moins.

Les solvants utilisés selon l'invention peuvent être utilisés seuls ou en combinaison. L'exécution la plus simple consiste dans l'emploi d'un seul solvant éventuellement sous la forme impure appelée couramment « de qualité technique ». De préférence, on choisit des solvants qui possèdent un point d'ébullition, mesuré à pression atmosphérique, compris entre 65 °C et 170 °C.

Les solvants tout particulièrement préférés sont les chloroéthanes et chloropropanes non substitués dont les atomes carbone porteurs d'au moins un atome de chlore sont également substitués par au moins un atome d'hydrogène. Dans cette catégorie les solvants qui donnent les meilleurs résultats sont le 1,2-dichloroéthane, le 1,1,2-trichloroéthane, le 1,1,2,2-tétrachloroéthane et le 1,2,3-trichloropropane. Les meilleurs résultats ont été obtenus avec le 1,2-dichloroéthane et le 1,1,2-trichloroéthane.

Les biomasses que l'on peut traiter selon l'invention peuvent être obtenues à partir de microorganismes d'origines diverses et notamment à partir de bactéries comme il a été décrit dans Angew. Chemie 1962, 74 Jahrg, Nr. 10 p. 342-346. La sélection des microorganismes se fait en général sur la base de la quantité relative de poly-β-hydroxybutyrates contenue dans le microorganisme ainsi qu'en fonction de la vitesse de croissance du microorganisme et de sa vitesse de synthèse des poly-β-hydroxybutyrates.

Les microorganismes, qu'ils soient cultivés artificiellement ou non, peuvent être traités directement par le solvant d'extraction dans leur milieu de culture. Une exécution préférée consiste toutefois à partir de microorganismes qui ont été, préalablement à l'extraction, séparés de leur milieu de culture. Cette séparation peut être effectuée par tous les moyens connus à cet effet. Une façon élégante de procéder consiste à centrifuger le milieu de culture de façon à pouvoir aisément séparer les microorganismes du milieu et à lyophiliser subséquemment les microorganismes isolés de façon à obtenir une biomasse qui peut être traitée aisément par le solvant d'extraction.

Le rapport entre la quantité de la biomasse mise en œuvre et le solvant d'extraction utilisé n'est pas critique en soi. On travaille en général avec des rapports en poids compris entre 1 à 1 et 1 à 100. Habituellement, on emploie des rapports compris entre 1 à 2 et 1 à 50 et enfin on opère de préférence avec des rapports compris entre 1 à 5 et 1 à 10. Le choix de ce rapport est influencé par divers paramètres tels que la nature de la biomasse à traiter, la température, le nombre d'extractions et le rendement souhaité par extraction. En outre, l'élimination des résidus cellulaires est d'autant plus facile qu'il y a moins de biomasse dans le milieu d'extraction.

La température à laquelle est effectuée l'extraction est choisie en fonction de la nature des chloroéthanes et chloropropanes utilisés. Les températures qui conviennent bien en général sont situées entre 50 °C et le point d'ébullition, mesuré sous pression atmosphérique, du solvant. On a constaté que les températures optimales d'opération se situent habituellement en dessous de 130 °C. Elles sont de

**0 014 490**

préférence comprises entre 60 et 90 °C. Dans le cas du 1,2-dichloroéthane, de bons résultats ont été obtenus entre 60 et 80 °C.

La pression à laquelle est effectuée l'extraction n'est pas critique et est généralement comprise entre 0,1 et 10 kg/cm² [98-9807 mbar].

L'opération d'extraction peut être réalisée dans tout appareillage conçu à cet effet. On peut effectuer l'opération en continu ou en discontinu avec des circulations des biomasses et des solvants d'extraction circulant dans le même sens ou dans des sens opposés.

Après l'opération d'extraction le solvant peut être débarrassé des composés non solubles dans le milieu tels que les membranes cellulaires de la biomasse extraite. Pour ce faire, on peut utiliser n'importe quel moyen connu. Habituellement cette opération est effectuée par une ou plusieurs filtrations.

Les poly-β-hydroxybutyrates extraits peuvent être séparés du milieu d'extraction par n'importe quelle méthode connue à cet effet. Ainsi, on peut les séparer par évaporation du solvant ou encore par simple addition d'un agent précipitant. Comme agents précipitants utilisables, on peut citer les composés non solvants des poly-β-hydroxybutyrates tels que l'éther de pétrole, les hydrocarbures aliphatiques non substitués, les solvants aromatiques dont le benzène ou encore les alcools aliphatiques. Des agents précipitants préférés sont les alcools aliphatiques et plus particulièrement, pour des raisons économiques, le méthanol et l'éthanol.

Les poly-β-hydroxybutyrates séparés selon l'invention peuvent être ensuite purifiés par un ou plusieurs lavages avec des non solvants tels que ceux cités ci-avant et être séchés, après quoi ils se présentent sous la forme d'une masse polymérique blanche. Les opérations de récupération et de purification se font habituellement à température ambiante.

Les poly-β-hydroxybutyrates sont des polymères ayant de nombreuses applications et notamment en chirurgie où ils peuvent être utilisés sous forme de fils car ils sont aisément stérilisables. En outre, ces polymères peuvent être mis en forme par les différentes techniques connues de moulage pour faire des prothèses. On peut encore les filer ou les extruder suivant les méthodes habituelles.

Les exemples suivants servent à illustrer l'invention.


### Exemple 1 (de comparaison)

Dans un ballon de 250 ml muni d'un agitateur et d'un réfrigérant à eau on introduit 100 ml de chloroforme que l'on porte à 60 °C. On introduit ensuite 16,0 g d'une biomasse séchée lyophilisée constituée de bactéries de la souche ALCALIGENES EUTROPHUS, délitée au mortier avant l'emploi, et dont la teneur en eau superficielle est de 23,2 g/kg. Après 1 heure, on prélève la solution et on filtre sous pression à chaud sur une toile en tissu afin d'éliminer les restes solides de la biomasse. Le polymère est précipité par addition d'éthanol en excès, à température ambiante, à la solution obtenue. Le précipité est filtré sous vide sur un filtre avec verre fritté et lavé avec de l'éthanol. Le polymère est ensuite séché jusqu'à poids constant sous vide. Le rendement de l'extraction est donné par le poids du produit extrait sur le poids de biomasse mis en œuvre et le poids moléculaire en poids de polymère est calculé à partir de la mesure de la viscosité intrinsèque d'une solution chloroformique selon la formule

$$(\eta) = 1,9 \cdot 10^{-2} M_W^{0,74} \qquad (\eta \text{ exprimé en ml/g}).$$

préconisée dans l'article paru dans Makromol. Chem. Vol. 176, 1975 p. 2662. Des valeurs obtenues sont rassemblées au Tableau 1 ci-après.


### Exemples 2 et 3 (de comparaison)

On répète l'exemple 1 mais en substituant respectivement du tétrachlorométhane (exemple 2) et du trichloroéthylène (exemple 3) au chloroforme. Les résultats obtenus sont rassemblés au Tableau 1.


### Exemples 4, 5 et 6

On opère de nouveau dans des conditions identiques à celles de l'exemple 1 mais on effectue l'opération d'extraction avec du 1,2-dichloroéthane (exemple 4), du 1,1,2-trichloroéthane (exemple 5) et du 1,1,2,2-tétrachloroéthane (exemple 6). Les résultats obtenus sont également repris au Tableau 1 ci-après.


### Exemple 7 (de comparaison)

L'exemple 1 est répété avec du chloroforme comme solvant d'extraction mais en opérant l'extraction pendant une durée de 3 heures. Les résultats obtenus sont repris au Tableau 1 ci-dessous.

3

Tableau 1

| Essai n° | 1 | 2 | 3 | 4 | 5 | 6 | 7 |
|---|---|---|---|---|---|---|---|
| Rendement % | 36,6 | 0 | 0 | 66,2 | 72,0 | 60,7 | 48,8 |
| Poids moléculaire × $10^{-3}$ | 887 | — | — | 902 | 920 | 905 | 576 |

On peut donc conclure des résultats rassemblés au Tableau 1 que les rendements observés par extraction selon l'invention sont nettement plus élevés qu'avec d'autres solvants halogénés dans les mêmes conditions opératoires. Ces rendements sont même plus élevés que ceux obtenus après une durée d'extraction triple avec un solvant halogéné typique de l'art antérieur.

En outre on peut voir que dans les exemples 4, 5, 6 les poids moléculaires des polymères séparés selon l'invention sont nettement supérieurs à ceux obtenus à la même température avec le chloroforme.

Exemple 8 (de comparaison)

On opère comme dans l'exemple 1 mais au lieu de travailler à 60 °C on effectue l'extraction à 120 °C et avec du carbonate de propylène au lieu de chloroforme. Le rendement observé et le poids moléculaire du polymère extrait sont repris au Tableau 2 ci-après.

Exemples 9 et 10

On travaille comme dans l'exemple 8 mais en remplaçant le carbonate de propylène par du 1,1,2,2-tétrachloroéthane (exemple 9) et par du 1,2,3-trichloropropane (exemple 10). Les résultats sont rassemblés au Tableau 2.

Tableau 2

| Essai n° | 8 | 9 | 10 |
|---|---|---|---|
| Rendement % | 67,6 | 65,5 | 63,7 |
| Poids moléculaire × $10^{-3}$ | 400 | 574 | 693 |

On peut donc déduire de ces résultats que l'invention permet d'obtenir, à la même température, des rendements d'extraction qui sont du même ordre de grandeur que ceux obtenus avec le carbonate de propylène tout en ne provoquant pas une dépolymérisation aussi importante du polymère extrait.

En outre, on peut déduire, de la comparaison des résultats des Tableaux 1, et 2, que l'invention permet d'obtenir des rendements d'extraction meilleurs ou tout au moins égaux à des températures de 60 °C comparés aux rendements des procédés connus à 120 °C sans provoquer une dépolymérisation aussi importante.

**Revendications**

1. Procédé de séparation de poly-β-hydroxybutyrates d'une biomasse par extraction au moyen de solvants halogénés liquides caractérisé en ce que les solvants halogénés liquides sont choisis parmi les chloroéthanes et les chloropropanes.

2. Procédé selon la revendication 1, caractérisé en ce que les solvants halogénés liquides sont choisis parmi les chloroéthanes et les chloropropanes ayant un point d'ébullition compris entre 65 °C et 170 °C.

3. Procédé selon la revendication 1 ou 2, caractérisé en ce que les solvants halogénés liquides sont choisis parmi les chloroéthanes et les chloropropanes possédant des atomes de carbone porteurs d'au moins un atome de chlore et également d'au moins un atome d'hydrogène.

4. Procédé selon l'une quelconque des revendications 1 à 3, caractérisé en ce que les solvants halogénés liquides sont choisis parmi le 1,2-dichloroéthane, le 1,1,2-trichloroéthane, le 1,1,2,2-tétrachloroéthane et le 1,2,3-trichloropropane.

5. Procédé selon l'une quelconque des revendications 1 à 4, caractérisé en ce que l'extraction est opérée entre 60 °C et 90 °C.

**Claims**

1. Process for separating poly-β-hydroxybutyrates from a biomass by extraction using liquid

4

**0 014 490**

halogenated solvents, characterised in that the liquid halogenated solvents are chosen from amongst chloroethanes and chloropropanes.

2. Process according to Claim 1, characterised in that the liquid halogenated solvents are chosen from amongst chloroethanes and chloropropanes having a boiling point of between 65 °C and 170 °C.

3. Process according to Claim 1 or 2, characterised in that the liquid halogenated solvents are chosen from amongst chloroethanes and chloropropanes which possess carbon atoms carrying at least one chlorine atom and also at least one hydrogen atom.

4. Process according to any one of Claims 1 to 3, characterised in that the liquid halogenated solvents are chosen from amongst 1,2-dichloroethane, 1,1,2-trichloroethane, 1,1,2,2-tetrachloroethane and 1,2,3-trichloropropane.

5. Process according to any one of Claims 1 to 4, characterised in that the extraction is carried out at between 60 °C and 90 °C.

**Ansprüche**

1. Verfahren zur Trennung von Poly-β-hydroxybutyraten aus einer Biomasse durch Extraktion mittels flüssiger halogenierter Lösungsmittel, dadurch gekennzeichnet, dass man die flüssigen halogenierten Lösungsmittel unter den Chlorethanen und den Chlorpropanen auswählt.

2. Verfahren nach Anspruch 1, dadurch gekennzeichnet, dass man die flüssigen halogenierten Lösungsmittel unter solchen Chlorethanen und Chlorpropanen auswählt, die einen Siedepunkt zwischen 65 °C und 170 °C aufweisen.

3. Verfahren nach Anspruch 1 oder 2, dadurch gekennzeichnet, dass man die flüssigen halogenierten Lösungsmittel unter solchen Chlorethanen und Chlorpropanen auswählt, die Kohlenstoffatome aufweisen, die mindestens ein Chloratom und gleichzeitig mindestens ein Wasserstoffatom tragen.

4. Verfahren nach einem der Ansprüche 1 bis 3, dadurch gekennzeichnet, dass man die flüssigen halogenierten Lösungsmittel unter 1,2-Dichlorethan, 1,1,2-Trichlorethan, 1,1,2,2-Tetrachlorethan und 1,2,3-Trichlorpropan auswählt.

5. Verfahren nach einem der Ansprüche 1 bis 4, dadurch gekennzeichnet, dass man die Extraktion zwischen 60 °C und 90 °C ausführt.